# EUROPEAN PATENT APPLICATION

(11) **EP 1 621 610 A1**
(43) Date of publication of application: **01.02.2006**
(21) Application number: 04726083.1
(22) Date of filing: 07.04.2004
(51) Int. Cl.: C12N 5/00, C12N 5/06, C12N 15/00

(54) **A METHOD OF PRODUCING NUCLEAR TRANSFER EMBRYONIC STEM CELL BY SOMATIC CELL NUCLEAR TRANSPLANTATION TECHNIQUE**

(30) Priority: 08.04.2003 CN 03113135
(71) Applicant: Southeast University, Jiangsu 210096 (CN); Polifarma (Nanjingo) Co.. Ltd., Nanjing, Jiangsu 210038 (CN)
(72) Inventor: MAO, Xi, Jiangsu 210096 (CN); MO, Yingru, Jiangsu 210038 (CN); PU, Yuepu, Jiangsu 210096 (CN); HUANG, Jinglong, Jiangsu 210038 (CN)
(74) Representative: Spera, Maria Caterina
(86) International application number: PCT/CN2004/000318
(87) International publication number: WO 2004/090119

(57) **Abstract**

The method of producing nuclear transfer embryonic stem cell by somatic cell nuclear transplantation technique is a method of producing cell by somatic cell nuclear transplantation technique, which comprises transferring the nucleus of nerve cell of adult animal into the cytoplast of enucleated embryonic stem cell of animal embryo using micromanipulator, reconstituting nuclear transfer embryonic stem cell having novel genotype, and culturing nuclear transfer embryonic stem cell to make it dissociate and proliferate up to 2⁸, which embryonic stem cell and nerve cell are allogenic, and the resultant cell is nuclear transfer embryonic stem cell having donor nuclear cell-nerve cell genotype. The present invention resolves not only the ethical problem of the use of embryonic stem cell in medicine, but also the problem of immunological rejection, which occurs in cell transplantation. It still resolves the cell cycle synchronization of donor nuclear cell and recipient nuclear cell and the source problem of recipient nuclear cell in somatic cell nuclear transplantation, so it would doubtless accelerate the development of human embryonic stem cell including the development of cell engineering and tissue engineering from this.

## Description

### Invention Field

The invention is about the method of generate cells by applying the cell nucleus transplantation technique. Generated cells are for cell-based therapy.

### Technical Background

For years, neurological injuries and diseases of the Central Nervous System (CNS) have posed a threat to human health. Though traditional pharmacological and surgical treatment options have proven to be effective, they are still limited in efficacy. There are still many difficulties to overcome such as the effectiveness of drugs, the proper surgical procedures to use, and the exact positioning of such procedures within the brain. Therefore, researchers have revolutionized a method to overcome these problems, cell therapy. This process involves transplanting groups of transformed cells, either identical or differentiated, into a specific area of the patient's brain or bone marrow. Certain diseases of the CNS can thus be treated with this technique. These transformed cells must exhibit the following characteristics:
1. These cells must be easily cultured and colonized in vitro;
2. They are able to divide into nervous cell, or obtain from the nervous system;
3. They are able to be cloned and induced to perform the intended task;
4. Regulator genes within these cells are able to selectively control the cell function;
5. After transplantation, the cells must have prolonged viability. After integration, cells must show compatibility with the existing receptor tissue.

The discovery and research of stem cells have provided a promising way for such cells to repair injuries of the CNS.

Stem cells are obtained from embryonic tissue, cultured in vitro, and develop into the precursor cells of their mature counterparts.

However, the final efficacy of the stem cell therapy is limited due to the potential rejection by the body's immune system, as well as strong ethics laws.

The purpose of this invention is to provide solutions to these two problems.

### The Invention Contents

The present invention is a method of generating embryonic stem cells by nucleus transplantation technique. Under micromanipulator, transplant nucleus which is taken from the adult animal's neuronal cells into animal's stem cell in which the nucleus has been removed, then it reconstitute and form a nucleus-transplanted embryonic stem cell which have new-generation gene. Culture the nucleus-transplanted embryonic stem cells to make them dissociate and proliferate up to 2⁸. The embryonic stem cells and neuronal cells are of same kind of cells. Obtained nucleus-transplanted embryonic stem cell have the characteristics of are donor cell and gene type neuronal cell. The procedures comprise the steps of:
1) Divide and clone embryonic stem cell which came from animal germ cell, and establish a system of animal embryonic stem cell;
2) Divide neural cell from animal grain tissue, then purifies it;
3) Remove nucleus from embryonic stem cell and take the nucleus from the neural cell;
4) Implant nucleus that take from the animal neural cell into inside the embryonic stem cell in which the nucleus has been removed, then reorganize a new embryonic stem cell;
5) Culture the nucleus-transplanted embryonic stem cell.

The embryonic stem cell comes from germ cells in gland of germ or the tissues near germ epithelium of non-human mammalian. The nucleus of neural cell comes from the neural cells divided from tissues of adult non-human mammalian. The non-human mammalian are mice or pigs.

### Detailed Description of the Invention

The present invention is a method of generating embryonic stem cells by nucleus transplantation technique. Under micromanipulator, transplant nucleus which is taken from the adult animal's neuronal cells into animal's stem cell in which the nucleus has been removed, then it reconstitute and form a nucleus-transplanted embryonic stem cell which have new-generation gene. Culture the nucleus-transplanted embryonic stem cells to make them dissociate and proliferate up to 2⁸. The embryonic stem cells and neuronal cells are of same kind of cells. Obtained nucleus-transplanted embryonic stem cells have the characteristics of the nucleus donor cell and gene type neuronal cell. The method comprises the step of:
1) Divide and clone embryonic stem cell which came from animal germ cell, and establish a system of animal embryonic stem cell;
2) Divide neural cell from animal grain tissue, then purifies it;
3) Remove nucleus from embryonic stem cell and take the nucleus from the neural cell;
4) Implant nucleus that take from the animal neural cell into inside the embryonic stem cell in which the nucleus has been removed, then reorganize a new embryonic stem cell;
5) Culture the nucleus-transferred embryonic stem cell.

The embryonic stem cell comes from germ cells in gland of germ or the tissues near germ epithelium of non-human mammalian. The nucleus of neural cell comes from the neural cells divided from tissues of adult non-human mammalian. The non-human mammalian are mice or pigs.

To achieve the goal of the invention, the detail procedures are that divide and clone embryonic stem cell which came from animal germ cell. Establish a system of animal embryonic stem cell. Stem cells are more easily extracted from animal germ cell lines. Thus these cells can be obtained from aborted fetuses than directly from female ovarian egg. Remove nucleus from embryonic stem cell and take the nucleus from the neural cell. Implant nucleus that take from the animal neural cell into inside the embryonic stem cell in which the nucleus has been removed, then reorganize a new embryonic stem cell. The reasons why neural cells are chosen as a nucleus donor cell are 1) to resolve the problem of the donor-receptor synchronization, because when a nucleus which is in S stage transfer to egg cell, the premature chromosome condensation (PCC) occurs. If the chromosome is disordered, the embryonic can not be alive. 2) Due to the gene's type of generated nucleus-transferred embryonic stem cells is the gene's type of the patient, it doesn't produce the rejection of immune system.

## Claims

1. A method for generating embryonic stem cells by nucleus transferring technique. It is **characterized** that under micromanipulator, transferring nucleus take from the adult animal's neuronal cells into animal's stem cell in which the nucleus has been removed, then reorganize it into a nucleus transferred embryonic stem cell that with new-generation gene. Culture the nucleus-transplanted embryonic stem cells to make them dissociate and proliferate up to 2⁸. The embryonic stem cells and neuronal cells are of same kind of cells. Obtained nucleus-transplanted embryonic stem cells have the characteristics of nucleus donor cell and gene type neuronal cell. The procedures comprise the steps of:
1) Divide and clone embryonic stem cell which came from animal germ cell, establish a system of animal embryonic stem cell;
2) Divide neural cell from animal grain tissue, then purifies it;
3) Remove nucleus from embryonic stem cell and take the nucleus from the neural cell;
4) Implant nucleus that take from the animal neural cell into inside the embryonic stem cell in which the nucleus has been removed, then reconstitute new embryonic stem cells;
5) Culture the nucleus-transferred embryonic stem cell.

2. The method of claim 1 wherein the embryonic stem cell comes from germ cells in gland of germ or tissues near germ epithelium of non-human mammalian.

3. The method of claim 1 wherein the nucleus of neural cell comes from the neural cells divided from tissues of adult non-human mammalian.

4. The method of claim 1 wherein using micromanipulator to remove the nucleus in vitro.

5. The method of claim 3, wherein the non-human mammalian are mice or pigs.
